(19) Europäisches Patentamt
European Patent Office
Office européen des brevets

(11) **EP 2 427 424 B1**

(12) **EUROPÄISCHE PATENTSCHRIFT**

(45) Veröffentlichungstag und Bekanntmachung des Hinweises auf die Patenterteilung:
**04.06.2014 Patentblatt 2014/23**

(21) Anmeldenummer: **10721405.8**

(22) Anmeldetag: **06.05.2010**

(51) Int Cl.:
*C07C 209/86* *(2006.01)*    *C07C 211/46* *(2006.01)*
*C07C 209/36* *(2006.01)*    *B01D 11/04* *(2006.01)*

(86) Internationale Anmeldenummer:
**PCT/EP2010/056209**

(87) Internationale Veröffentlichungsnummer:
**WO 2010/128118 (11.11.2010 Gazette 2010/45)**

(54) **VERFAHREN ZUR HERSTELLUNG EINES AROMATISCHEN AMINS SOWIE VORRICHTUNG DAZU**

METHOD FOR PRODUCING AN AROMATIC AMINE AND DEVICE THEREFOR

PROCÉDÉ DE PRÉPARATION D'UNE AMINE AROMATIQUE ET DISPOSITIF ASSOCIÉ

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **07.05.2009 EP 09159617**

(43) Veröffentlichungstag der Anmeldung:
**14.03.2012 Patentblatt 2012/11**

(73) Patentinhaber: **BASF SE**
**67056 Ludwigshafen (DE)**

(72) Erfinder:
• **STEINMETZ, Tilmann**
**67269 Grünstadt (DE)**
• **NILLES, Michael**
**67240 Bobenheim-Roxheim (DE)**
• **KÖNIGSMANN, Lucia**
**70197 Stuttgart (DE)**
• **HEUßLER, Andreas**
**67454 Haßloch (DE)**
• **ZAFRED, Nikolaus**
**67059 Ludwigshafen (DE)**

(56) Entgegenhaltungen:
**DE-A1-102006 008 000**    **GB-A- 666 544**
**GB-A- 2 352 715**

EP 2 427 424 B1

**Beschreibung**

Die Erfindung betrifft ein Verfahren zur Herstellung mindestens eines aromatischen Amins durch Extraktion eines Wasser und das mindestens eine aromatische Amin enthaltenden Flüssigkeitsgemischs mit mindestens einem Nitroaromaten in einer Extraktionskolonne, wobei ein im Wesentlichen Wasser enthaltender Raffinatstrom und ein das mindestens eine Nitroaromat und das aromatische Anilin enthaltender Extraktstrom gebildet werden. Die Erfindung betrifft weiterhin ein Verfahren zur Herstellung von aromatischen Aminen durch katalytische Hydrierung des korrespondierenden Nitroaromaten und eine Vorrichtung zur Trennung eines Wasser und eines mindestens ein aromatisches Amin enthaltenden Flüssigkeitsgemischs durch Extraktion mit mindestens einem Nitroaromaten, eine Extraktionskolonne umfassend.

Das durch die Extraktion zu trennende Wasser und das aromatische Amin enthaltende Flüssigkeitsgemisch entstammt im Allgemeinen einem Verfahren zur Herstellung von aromatischen Aminen durch Hydrierung des korrespondierenden Nitroaromaten in Gegenwart eines heterogenen Katalysators. Ein solches Verfahren zur Herstellung von aromatischen Aminen ist zum Beispiel in EP-B 0 507 118 beschrieben.

Vorzugsweise wird der zur Herstellung des aromatischen Amins eingesetzte Nitroaromat als Solvent in der Extraktion zur Gewinnung des aromatischen Armins eingesetzt. Bei der Extraktion bildet sich eine das aromatische Amin und den Nitroaromaten enthaltende Extraktphase, die in einer nachfolgenden Trennstufe, beispielsweise einer Destillation, in das aromatische Amin als Wertprodukt und einen im Wesentlichen den Nitroaromaten enthaltenden Strom getrennt wird. Der so abgetrennte Nitroaromat kann dann als Edukt ebenfalls in den Reaktor zur Herstellung des aromatischen Amins geleitet werden.

Ein Verfahren zur Gewinnung eines aromatischen Amins aus einer wässrigen Phase durch Extraktion des aromatischen Amins mit einem korrespondierenden Nitroaromaten ist zum Beispiel aus DE-A 10 2006 008 000 bekannt. Als Solvent für die Extraktion wird dabei vorzugsweise der zur Herstellung des aromatischen Amins eingesetzte Nitroaromat eingesetzt. Bei der Extraktion löst sich das aromatische Amin im Nitroaromaten und kann anschließend durch eine destillative Trennung aus diesem entfernt werden.

Ein weiteres Verfahren zur Rückgewinnung aromatischer Amine ist aus GB-A 2 352 715 bekannt.

Für die Extraktion werden gängige, dem Fachmann bekannte Extraktionsvorrichtungen, beispielsweise Mixer/Settler oder Extraktionskolonnen, eingesetzt. Nachteil gängiger Extraktionskolonnen ist jedoch, dass diese zur Entfaltung der abgesicherten Trennleistung zum Betrieb Flüssigkeitsbeladungen in einer bestimmten Größe benötigen. Wenn jedoch große Schwankungen beim Feed auftreten, so kann dies zu einer Ausweitung von Teillastwirbeln führen, was mit einer zu geringen Flüssigkeitsbeladung und einer Verschlechterung der Extraktionsleistung bis hin zum Nichterreichen der Raffinatkonzentration einhergeht. Dies hat bei Einsatz von Anilin als aromatischem Amin eine Anilin-Schwarz-Bildung in der nachfolgenden Stufe zur Folge.

Eine Extraktionskolonne ist zum Beispiel in GB-A 666 544 offenbart.

Aufgabe der vorliegenden Erfindung ist es, ein Verfahren und eine Vorrichtung zur Gewinnung von aromatischem Amin aus einem Wasser und das mindestens eine aromatische Amin enthaltenden Flüssigkeitsgemisch durch Extraktion bereitzustellen, die auch bei stark schwankenden Volumenströmen des zu trennenden Wasser und aromatischem Amin enthaltenden Flüssigkeitsgemischs ohne Trennleistungsverlust betrieben werden können.

Gelöst wird die Aufgabe durch ein Verfahren zur Gewinnung mindestens eines aromatischen Amins aus einem Wasser und das mindestens eine aromatische Amin enthaltenden Flüssigkeitsgemisch durch Extraktion mit mindestens einem Nitroaromaten in einer Extraktionskolonne, wobei ein im Wesentlichen Wasser enthaltender Raffinatstrom und ein den mindestens einen Nitroaromaten und das aromatische Amin enthaltender Extraktstrom gebildet werden, wobei die Extraktionskolonne durch eine vertikale Trennwand in zwei Bereiche getrennt wird und bei einer zu trennenden Flüssigkeitsmenge, die kleiner ist als eine minimale Querschnittsbelastung der gesamten Extraktionskolonne, nur einem der durch die vertikale Trennwand getrennten Bereiche der Extraktionskolonne das zu trennende Flüssigkeitsgemisch zugeführt wird.

"Im Wesentlichen Wasser enthaltender Raffinatstrom" im Sinne der vorliegenden Erfindung bedeutet, dass der Raffinatstrom mindestens 98 Gew.-% Wasser, mehr bevorzugt mindestens 99,5 Gew.-% Wasser, insbesondere mindestens 99,99 Gew.-% Wasser enthält.

Die obere Betriebsgrenze einer Extraktionskolonne wird durch den Flutpunkt bestimmt und legt die maximale Kolonnenbelastung fest. Die maximale Kolonnenbelastung liegt dabei üblicherweise 75 bis 80 % unterhalb der Flutpunktbelastung, die den Betriebspunkt bei maximaler Querschnittsbelastung der Extraktionskolonne widerspiegelt. Eine höhere maximale Kolonnenbelastung wird üblicherweise nicht angesetzt, da das Risiko bei Schwankungen der Zulaufströme, Temperaturen oder Konzentrationen zu groß ist, einen instabilen Betriebspunkt zu erreichen.

Die unterste Lastgrenze einer Extraktionskolonne liegt üblicherweise bei etwa 50 % Teillast, was üblicherweise einer Belastung von etwa 40 % der Flutbelastung entspricht. Bei solch niedrigen Lasten nimmt jedoch bekannterweise die Rückvermischung in Füllkörperkolonnen stark zu. Zudem haben die Flüssigkeitsverteiler, mit denen die Flüssigkeiten in die Extraktionskolonne zugegeben werden, nur einen engen Arbeitsbereich, in dem die benötigten Tropfengrößen erzeugt werden können. Durch die erfindungsgemäße vertikale Trennwand kann der Querschnitt der Extraktionskolonne verkleinert werden. Üblicherweise liegt die vertikale Trennwand in der Kolonnenmitte, so dass der Querschnitt halbiert wird. Hierdurch ist ein Betrieb der Extraktionskolonne mit einer sehr viel niedrigeren Last möglich. Bei Betrieb nur eines der durch die vertikale Trennwand getrennten Bereiche wird bereits bei halber Last eine der maximalen Kolonnenbelastung der gesamten Extraktionskolonne vergleichbare Querschnittsbelastung erreicht. Hierdurch lässt sich der Betriebsbereich der Extraktibnskolonne gegenüber einer Extraktionskolonne ohne vertikale Trennwand stark erweitern.

Durch die vertikale Trennwand in der Extraktionskolonne ist es somit möglich, auch bei einem kleinen zu trennenden Flüssigkeitsstrom eine Extraktion ohne großen Trennleistungsverlust durchführen zu können. So wird zum Beispiel auch eine Vergrößerung auftretender Wirbel in der Extraktionskolonne durch die vertikale Trennwand verhindert und von der Ausdehnung auf den Querschnitt zwischen Kolonnenwand und vertikaler Trennwand reduziert. Die sich bei Teillast der Extraktionskolonne ausbildenden Wirbel in Größe des Durchmessers der Kolonne führen zu einer Vergrößerung der Rückvermischung. Die Rückvermischung verschlechtert im Allgemeinen die auf die Kolonnenhöhe bezogene Trennleistung, was dazu führt, dass die Höhe der Kolonne vergrößert werden muss. Durch die vertikale Trennwand lässt sich die Bauhöhe der Extraktionskolonne für einen sehr breiten Lastbereich geringer wählen als die notwendige Bauhöhe für den gleichen Lastbereich ohne die vertikale Trennwand.

Ein weiterer Vorteil des erfindungsgemäßen Verfahrens ist es, dass auch bei Betrieb von zwei Reaktoren zur Herstellung des mindestens einen aromatischen Amins nur eine Extraktionskolonne benötigt wird, um das bei der Reaktion entstehende, Wasser und aromatisches Amin enthaltende Flüssigkeitsgemisch aufzubereiten. Es wird auch bei Betrieb nur einer der Reaktoren, die mit der Extraktionskolonne verbunden sind, eine ausreichende Trennwirkung für ein hinreichend reines aromatisches Amin als Produkt erzielt.

Als Extraktionskolonne kann jede beliebige, dem Fachmann bekannte Extraktionskolonne verwendet werden. Geeignete Kolonnen weisen Einbauten, üblicherweise in Form von strukturierten oder unstruktierten Packungen auf. Insbesondere werden unstrukturierte Packungen, im Allgemeinen Füllkörper eingesetzt. Als Füllkörper eignen sich dabei alle beliebigen, dem Fachmann bekannten Füllkörper. Insbesondere geeignete Füllkörper sind zum Beispiel Pallringe.

Die strukturierte Packung in der Extraktionskolonne ist dabei zwischen dem ersten Zulauf am Kopf und dem zweiten Zulauf am unteren Ende der Kolonne positioniert. Durch den Einsatz der Packungen wird eine gleichmäßige Flüssigkeitsverteilung in der Extraktionskolonne, bzw. in dem betriebenen Bereich der Extraktionskolonne erzielt.

In einer ersten Ausführungsform endet die vertikale Trennwand unterhalb dem Flüssigkeitsspiegel in der Extraktionskolonne und oberhalb einer ersten Zufuhrvorrichtung am Kopf der Extraktionskolonne. Dadurch, dass die vertikale Trennwand unterhalb dem Flüssigkeitsspiegel in der Extraktionskolonne endet, wird die Flüssigkeit aus beiden durch die vertikale Trennwand getrennten Bereiche der Extraktionskolonne in einem gemeinsamen Kopf gesammelt. Dies hat den Vorteil, dass nur eine Füllstandsmessung am Kopf der Kolonne enthalten sein muss. Auch bei Betrieb nur eines Teilbereichs der Extraktionskolonne kann so unabhängig vom betriebenen Bereich eine Regelung mit nur einer Flüssigkeitsstandsmessung durchgeführt werden.

Weiterhin ist es vorteilhaft, wenn die vertikale Trennwand die sich ausbildende Phasengrenze durchdringt. Dabei ist es weiterhin bevorzugt, wenn der Sumpf der Extraktionskolonne ebenfalls für beide durch die vertikale Trennwand getrennten Bereiche nicht getrennt wird. Hierdurch ist es ebenfalls ausreichend, sämtliche für die Regelung der Extraktion notwendigen Messvorrichtungen am Sumpf der Kolonne nur in einfacher Ausführung vorzusehen. Es müssen nicht beide Teilbereiche getrennt betrachtet werden.

Dass die vertikale Trennwand in die abzutrennende Flüssigphase im Sumpf der Extraktionskolonne hineinragt bedeutet, dass das untere Ende der vertikalen Trennwand unterhalb einer Phasengrenze zwischen der abzutrennenden Flüssigphase und der alle in der Extraktion eingesetzten Flüssigkeiten enthaltenden Phase liegt.

Um bei der nicht durchgehenden vertikalen Trennwand nur einen Bereich der Extraktionskolonne zu betreiben, ist es vorteilhaft, im zweiten Bereich Flüssigkeit stehen zu lassen.

Um zu vermeiden, dass aufgrund der herrschenden hydrostatischen Druckdifferenz auf Basis der unterschiedlichen mittleren Dichten in den beiden Bereichen der Extraktionskolonne, die durch die vertikale Trennwand gebildet werden, eine Schlaufenströmung um die Trennwand zwischen dem aktiven Bereich und dem ruhenden Bereich entsteht, ist es notwendig, dass die vertikale Trennwand soweit in die abzutrennende Flüssigphase im Sumpf der Extraktionskolonne hineinragt, dass die unterschiedliche Lage der Phasengrenze zwischen der abzutrennenden Flüssigphase und dem Extraktionsgemisch, das alle Komponenten enthält, nicht zu einer Unter- bzw. Überströmung der vertikaler Trennwand mit der kontinuierlichen Phase führt.

Die Höhendifferenz der Phasengrenzen $h_{trenn}$ lässt sich über die Dichten der Phasen, dem erwarteten Hold-Up-$\phi$ und der Standhöhe der zweiphasig betriebenen Kolonnenhälfte $H_{akt}$ wie folgt abschätzen:

$$h_{trenn} = \phi \cdot \frac{\left|(\rho_c - \rho_d)\right|}{\rho_d} \cdot H_{akt} \, .$$

Eine Schlaufenströmung um die vertikale Trennwand kann alternativ auch dadurch verhindert werden, dass die vertikale Trennwand zum Beispiel entweder mit dem Boden am Sumpf der Extraktionskolonne abschließt und so auch den Sumpf der Extraktionskolonne in zwei Bereiche teilt oder bis über den maximalen Flüssigkeitsspiegel der Extraktionskolonne am Kopf hinausragt und zum Beispiel auch mit dem den Kopf verschließenden Deckel der Extraktionskolonne verbunden ist. Alternativ ist es auch möglich, dass die vertikale Trennwand die gesamte Extraktionskolonne in zwei separate Bereiche trennt. Wenn entweder der Sumpf oder der Kopf oder auch Sumpf und Kopf der Extraktionskolonne durch die vertikale Trennwand in zwei separate Bereiche getrennt werden, so ist es notwendig, jeweils separate Zu- und Abläufe in die einzelnen Teilbereiche der Extraktionskolonne vorzusehen. So müssen bei einer Trennung des Sumpfes in zwei Bereiche zwei separate Sumpfabläufe zur Entnahme der am Sumpf der Flüssigkeit abzutrennenden Flüssigphase vorgesehen sein. Bei einer Trennung der im Kopf der Extraktionskolonne enthaltenden Flüssigphase ist es notwendig, in jedem der Bereiche einen Entnahmestutzen vorzusehen, um die sich am Kopf der Extraktionskolonne sammelnde Flüssigkeit zu entnehmen. Auch müssen bei einer entsprechenden Trennung in jeweils separate Bereiche in jedem Bereich eigene Messvorrichtungen vorgesehen sein, um die Extraktion steuern und regeln zu können. Aus diesem Grund ist es bevorzugt, wenn sowohl die am Sumpf der Kolonne enthaltende Flüssigkeit als auch die am Kopf der Kolonne enthaltende Flüssigkeit nicht durch die vertikale Trennwand getrennt werden.

Aufgrund der Dichteunterschiede der Flüssigkeiten wird die Extraktionskolonne üblicherweise so betrieben, dass am Kopf der Kolonne ein im Wesentlichen Wasser enthaltender Raffinatstrom entnommen wird. "Im Wesentlichen Wasser enthaltend" bedeutet im Rahmen der vorliegenden Erfindung, dass der am Kopf der Kolonne entnommene Raffinatstrom mindestens 98 Gew.-%, bevorzugt mindestens 99,5 Gew.-% und insbesondere mindestens 99,99 Gew.-% Wasser enthält.

Entsprechend ist die abzutrennende Flüssigphase im Sumpf der Extraktionskolonne eine im Wesentlichen wasserfreie, das mindestens eine Nitroaromat und das mindestens eine aromatische Amin enthaltende Extraktphase. Im Wesentlichen wasserfrei bedeutet im Sinne der vorliegenden Erfindung, dass die Extraktphase fremdphasenfrei ist oder maximal 1 Gew.-% einer Fremdphase enthält. Wenn die Extraktphase fremdphasenfrei ist, bedeutet dies, dass der Anteil an Wasser in der Extraktphase maximal dem Anteil entspricht, der sich aufgrund von Temperatur und Druck in der Extraktphase löst.

Um die Extraktionskolonne in Abhängigkeit von der Menge des zu trennenden Wasser und aromatisches Amin enthaltenden Flüssigkeitsgemischs entweder vollständig oder nur in einem der durch die vertikale Trennwand getrennten Bereiche zu betreiben, mündet in jeden der durch die vertikale Trennwand getrennten Bereiche ein erster Zulauf im oberen Bereich der Extraktionskolonne und ein zweiter Zulauf am unteren Ende der Kolonne oberhalb des Sumpfs.

Durch den ersten Zulauf am Kopf der Kolonne wird üblicherweise der als Solvent dienende Nitroaromat zugeführt. Über den zweiten Zulauf am unteren Ende der Kolonne oberhalb des Sumpfs wird das zu trennende Wasser und das mindestens eine aromatische Amin enthaltende Flüssigkeitsgemisch als Feed zugeführt. Durch die Zufuhr von Feed und Solvent an gegenüberliegenden Enden der Kolonne wird eine Gegenstromführung von Solvent und Feed erzielt.

Um die einzelnen Bereiche der Extraktionskolonne getrennt betreiben zu können, ist es weiterhin notwendig, die getrennten Zuläufe in die einzelnen Bereiche der Extraktionskolonne separat mit den in die Extraktionskolonne einzuleitenden Flüssigkeiten beaufschlagen zu können. Hierzu ist es zum Beispiel möglich, die einzelnen Zuläufe jeweils durch geeignete Ventile verschließen zu können. Vorzugsweise sind die Ventile dabei in Abhängigkeit von der insgesamt zu trennenden Menge an Wasser und dem mindestens einen aromatischen Amin enthaltenden Flüssigkeitsgemisch steuerbar.

Um zu vermeiden, dass bei längerem Betrieb nur eines Bereichs der Extraktionskolonne über lange Zeit im zweiten Bereich der Extraktionskolonne die gleiche Flüssigkeit steht, ist es weiterhin bevorzugt, in diesem Fall in regelmäßigen Abständen den Betrieb zwischen den Bereichen zu wechseln.

Der zur Gewinnung des aromatischen Amins eingesetzte Nitroaromat ist vorzugsweise Nitrobenzol und das aromatische Amin ist vorzugsweise Anilin.

Die Erfindung betrifft weiterhin ein Verfahren zur Herstellung von aromatischen Aminen durch katalytische Hydrierung des korrespondierenden Nitroaromaten, welches folgende Schritte umfasst:

(a) Umsetzen eines den in Nitroaromaten und Wasserstoff enthaltenden Reaktionsgemischs in Gegenwart eines heterogenen Katalysators zu einem das aromatische Amin, Wasser und nicht umgesetzten Wasserstoff enthaltenden Produktgemisch in einer Gleichgewichtsreaktion,

(b) Entfernen des Wasserstoffs aus dem Produktgemisch,

(c) Entwässern des Produktgemischs, wobei ein im Wesentlichen wasserfreies aromatisches Amin als Produkt und ein Wasser und Rückstände des aromatischen Amins enthaltendes Flüssigkeitsgemisch erhalten werden,

(d) Trennen des in Schritt (c) erhaltenen Wasser und Rückstände des aromatischen Amins enthaltenden Flüssigkeitsgemischs in einem wie vorstehend beschriebenen Verfahren durch Extraktion mit einem Nitroaromaten in einen im Wesentlichen Wasser enthaltenden Raffinatstrom und einen den Nitroaromaten und das aromatische Anilin enthaltenden Extraktstrom.

Durch Einsatz des vorstehend beschriebenen Verfahrens zur Extraktion des das Wasser und das aromatische Amin enthaltenden Flüssigkeitsgemischs in Schritt (d) ist es möglich, in Abhängigkeit vom benötigten aromatischen Amin das Herstellungsverfahren in unterschiedlicher Auslastung zu führen. Auch ist es möglich, zum Beispiel zwei separate Vorrichtungen zur Durchführung des Herstellungsverfahrens mit nur einer Extraktionskolonne zu verbinden und alternativ nur eine der beiden Reaktionstrassen oder beide Reaktionsstraßen zu betreiben.

Als Reaktor, der zur Herstellung des aromatischen Amins eingesetzt wird, eignet sich jeder beliebige, dem Fachmann bekannte und für die Hydrierung von Nitroaromat zu aromatischem Amin geeignete Reaktor. Üblicherweise eingesetzte Reaktoren sind zum Beispiel Wirbelschichtreaktoren, in denen der für die Reaktion eingesetzte heterogene Katalysator als Wirbelgranulat vorliegt. In einem solchen Wirbelschichtreaktor wird die Reaktion in Gasphase durchgeführt. Hierzu werden vorzugsweise sowohl der Wasserstoff als auch der Nitroaromat gasförmig zugegeben. Alternativ ist es jedoch auch möglich, den Nitroaromaten flüssig zuzugeben und im Reaktor zu verdampfen. Bevorzugt ist jedoch eine gasförmige Zugabe.

Zur gezielten Steuerung der Reaktionstemperatur ist es möglich, im Wirbelschichtreaktor einen Wärmeübertrager vorzusehen, der endothermen Reaktionen Wärme zuführen bzw. bei exothermen Reaktionen Wärme abführen kann. Der Wärmeübertrager kann dabei plattenförmig oder rohrförmig ausgebildet sein und im Wirbelschichtreaktor vertikal, horizontal oder geneigt angeordnet sein. Die Rohre oder Platten des Wärmeübertragers werden dabei von einem geeigneten Wärmeträger durchströmt.

Neben einem Wirbelschichtreaktor eignet sich jedoch zum Beispiel auch ein Rohrreaktor oder eine Reaktionskolonne, die den Katalysator enthalten. In diesem Fall ist der Katalysator vorzugsweise in Form einer Packung in die Reaktionskolonne oder den Rohrreaktor eingebaut.

Als Katalysatoren für die Hydrierung des Nitroaromaten zum aromatischen Amin eignen sich die bekannten, partikelförmigen, geträgerten oder nicht-geträgerten Katalysatoren, die zur Hydrierung von aromatischen Aminen eingesetzt werden. Insbesondere geeignet sind Katalysatoren, die Schwermetalle der ersten und/oder der fünften bis achten Gruppe des Periodensystems enthalten. Bevorzugt enthält der Katalysator eins oder mehrere der Elemente Kupfer, Palladium, Molybdän, Wolfram, Nickel und Kobalt.

Um eine möglichst vollständige Umsetzung des eingesetzten Nitroaromaten zu erhalten, ist es bevorzugt, die Reaktion mit einem Wasserstoffüberschuss zu betreiben. Da das bei der Reaktion entstehende Produktgemisch in der Regel gasförmig vorliegt und im Allgemeinen noch den nicht reagierten Wasserstoff enthält, ist es notwendig, in einem nächsten Schritt zunächst den Wasserstoff aus dem Produktgemisch zu entfernen. Bevorzugt wird hierzu ein Kondensator eingesetzt, in dem das aromatische Amin und das Wasser auskondensieren. Die gasförmigen Bestandteile, insbesondere der Wasserstoff werden abgetrennt. Der bei der Kondensation des aromatischen Amins und des Wassers abgetrennte weiterhin gasförmig vorliegende Wasserstoff wird vorzugsweise zurück in die Reaktion geführt. Um gegebenenfalls enthaltene gasförmige Inertstoffe aus dem Prozess zu entfernen, ist es weiterhin möglich, einen Teil des nicht abreagierten Wasserstoffs aus dem Prozess auszuschleusen. Dieser kann zum Beispiel gereinigt werden und anschließend ebenfalls in die Reaktion zurückgeführt werden. Alternativ ist es jedoch zum Beispiel auch möglich, den Wasserstoff beispielsweise als Brennstoff zu nutzen.

Nach dem Entfernen des Wasserstoffs aus dem das aromatische Amin, Wasser und den nicht umgesetzten Wasserstoff enthaltenden Produktgemisch wird das verbleibende aromatisches Amin und Wasser enthaltende Produktgemisch zur Gewinnung des aromatischen Amins als Produkt aufgearbeitet. Hierzu wird das Produktgemisch entwässert, wobei ein im Wesentlichen wasserfreies aromatisches Amin als Produkt und ein Wasser und Rückstände des aromatischen Amins enthaltendes Flüssigkeitsgemisch erhalten werden. Die Entwässerung erfolgt zum Beispiel durch eine Flüssig/Flüssig-Phasentrennung. Die Phasentrennung kann dabei in jeder beliebigen, zur Flüssig/Flüssig-Phasentrennung geeigneten Vorrichtung durchgeführt werden. Neben einer Flüssig/Flüssig-Phasentrennung lässt sich jedoch auch jedes beliebige andere geeignete, dem Fachmann bekannte Verfahren zur Entwässerung des aromatisches Amin und Wasser enthaltenden Produktgemischs einsetzen.

Durch die Entwässerung entstehen ein Flüssigkeitsgemisch, das Wasser und Rückstände des aromatischen Amins enthält, und eine organische Phase, die das gewünschte aromatische Amin als Rohprodukt enthält. Die Menge des aromatischen Amins in dem das Wasser und die Rückstände des aromatischen Amins enthaltenden Flüssigkeitsgemisch ist dabei abhängig von der druck- und temperaturabhängigen Löslichkeit des entsprechenden aromatischen Amins in Wasser. Bezogen auf Anilin als aromatisches Amin enthält das Wasser und Rückstände des aromatischen Amins enthaltende Flüssigkeitsgemisch bei Atmosphärendruck und Raumtemperatur im Allgemeinen 0,5 bis 15 Gew.-%, bevorzugt 1 bis 10 Gew.-%, besonders bevorzugt 3 bis 5 Gew.-% Anilin, sowie gegebenenfalls geringe Mengen an Nebenprodukten.

Die das aromatische Amin als Rohprodukt enthaltende Phase wird zur Gewinnung eines reinen aromatischen Amins im Allgemeinen nach dem Fachmann bekannten Verfahren gereinigt. Hierzu ist es zum Beispiel möglich, das das aromatische Amin enthaltende Produkt in einer Destillation weiter aufzuarbeiten. Hierbei wird üblicherweise das aromatische Amin als Kopfstrom gewonnen und im Produkt enthaltene Rückstände werden am Sumpf der Kolonne entfernt.

Die Destillation kann dabei in jeder beliebigen, dem Fachmann bekannten Vorrichtung zur Destillation durchgeführt werden. Üblicherweise wird hierzu eine Destillationskolonne eingesetzt. Geeignete Destillationskolonnen weisen im Allgemeinen Einbauten, zum Beispiel in Form von Böden, strukturierten Packungen oder unstrukturierten Packungen auf. Als Böden in einer Destillationskolonne eignen sich zum Beispiel Glockenböden, Siebböden oder Ventilböden. Üblicherweise eingesetzte strukturierte Packungen sind im Handel erhältlich und werden zum Beispiel unter der Bezeichnung Mellapak® von der Firma Sulzer vertrieben. Für unstrukturierte Packungen werden im Allgemeinen Füllkörper eingesetzt. Damit die Füllkörper in der Destillationskolonne verbleiben und nicht durch diese hindurchfallen, wird üblicherweise ein Gitterboden eingesetzt, auf dem die Füllkörper aufliegen. Geeignete Füllkörper, die in der Destillationskolonne eingesetzt werden können, sind dem Fachmann bekannt. Üblicherweise eingesetzte Füllkörper sind zum Beispiel Pallringe, Raschigringe, Berl-Sättel oder Intalox-Sättel. Aber auch jede beliebige andere Form von Füllkörpern, die dem Fachmann bekannt ist, kann eingesetzt werden. Übliche Materialien aus denen die Füllkörper gefertigt sind, sind zum Beispiel Metalle, Keramiken oder Kunststoffe. Bei der Auswahl des Materials ist lediglich darauf zu achten, dass dieses gegenüber den in der Destillation zu trennenden Medien inert ist.

Die gewünschte Produktreinheit des im Wesentlichen das aromatische Amin enthaltenden Produktstroms lässt sich durch eine geeignete Destillationsführung erzielen. So ist es zum Beispiel möglich, eine mehrstufige Destillation durchzuführen. Alternativ ist es auch möglich, die Destillationskolonne mit entsprechend vielen theoretischen Böden zu gestalten. Eine weitere Möglichkeit, die Produktreinheit des Produktstroms zu erhöhen ist es, am Sumpf der Destillationskolonne einen Verdampfer vorzusehen und zumindest einen Teil des Sumpfstroms zu verdampfen und in die Destillationskolonne zurückzuführen.

Aus dem das Wasser und Rückstände des aromatischen Amins enthaltenden Flüssigkeitsgemisch wird das aromatische Amin durch das vorstehend beschriebene Verfahren extrahiert. Die Extraktion erfolgt dabei mit einem Nitroaromaten, insbesondere mit dem als Edukt für die Reaktion eingesetzten Nitroaromaten. Bei der Extraktion löst sich das aromatische Amin im Nitroaromaten und bildet eine den Nitroaromaten und aromatische Amin enthaltende Extraktphase. Das vom aromatischen Amin befreite Wasser tritt als Raffinatphase aus.

In einer bevorzugten Ausführungsform des erfindungsgemäßen Verfahrens zur Herstellung von aromatischen Aminen ist der in Schritt (d) als Solvent eingesetzte Nitroaromat der zur Herstellung des aromatischen Amins eingesetzte Nitroaromat. Hierdurch wird vermieden, dass bei der Extraktion eine Verunreinigung durch einen weiteren Nitroaromaten erfolgt. Es ist nicht notwendig, einen zusätzlichen Stoff einzusetzen.

Ein weiterer Vorteil der Verwendung des zur Herstellung des aromatischen Amins eingesetzten Nitroaromaten als Solvent in der Extraktion ist, dass nach der Extraktion der Nitroaromat als Edukt in die Reaktion zur Herstellung des aromatischen Amins in Schritt (a) zugeführt werden kann. Hierzu ist es bevorzugt, wenn der in Schritt (d) erhaltene Extraktstrom in den Reaktor zurückgeführt wird. Auf diese Weise wird auch das im Extraktstrom enthaltene aromatische Amin in den Reaktor zurückgeführt und gelangt so erneut in die Entwässerung und kann als Produkt gewonnen werden. Auf diese Weise wird das bei der Extraktion abgetrennte aromatische Amin ebenfalls als Wertprodukt im Prozess gewonnen und nicht mit dem Nitroaromaten als Extraktionsmittel aus dem Prozess ausgeschleust. Die Rückführung in den Reaktor hat zudem den Vorteil, dass keine weitere Aufarbeitung des Extraktstroms notwendig ist.

Die weitere Aufgabe wird gelöst durch eine Vorrichtung zur Trennung eines Wasser und mindestens ein aromatisches Amin enthaltenden Flüssigkeitsgemischs durch Extraktion mit mindestens einem Nitroaromaten, umfassend eine Extraktionskolonne, wobei die Extraktionskolonne durch eine vertikale Trennwand in zwei Bereiche getrennt wird, und weiterhin Mittel zur Steuerung vorgesehen sind, mit denen die Extraktion so geregelt werden kann, dass bei einer zu trennenden Flüssigkeitsmenge, die kleiner ist als die minimale Querschnittsbelastung der gesamten Extraktionskolonne, nur einem der durch die Trennwand getrennten Bereiche der Extraktionskolonne das zu trennende Flüssigkeitsgemisch zugeführt wird.

Als Mittel zur Steuerung werden insbesondere geeignete Sensoren zur Erfassung von Betriebszuständen, beispielsweise Füllstandsmesser, Durchflussmesser, Temperatursensoren und Drucksensoren sowie geeignete Regler zur Regelung von Ventilen oder anderen steuer- und regelbaren Einheiten verstanden. Üblicherweise ist auch eine Regelungseinheit umfasst, über die manuell in den Prozess eingegriffen werden kann und die relevante Parameter anzeigt. Größen, die erfasst werden, sind insbesondere der Flüssigkeitsstand in der Extraktionskolonne, die Lage der Phasengrenze der abzutrennenden Flüssigkeit, Durchflüsse der zugeführten und abgeführten Komponenten sowie das Verhältnis von zugeführtem zu trennendem Flüssigkeitsgemisch zu zugeführtem Solvent. Mit diesen Größen lassen sich zum Beispiel der Flüssigkeitsstand, Durchflussmengen der zugeführten Flüssigkeiten und Massen- bzw. Volumenströme der entnommenen Flüssigkeiten einstellen. Anhand der Menge der zugeführten Flüssigkeiten kann zudem beurteilt werden ob nur einer oder beide der durch die vertikale Trennwand getrennten Bereiche der Extraktionskolonne betrieben werden sollen.

Die vertikale Trennwand in der Extraktionskolonne erlaubt es, jeweils nur einen der durch die Trennwand geteilten Bereiche zu betreiben, während der andere Bereich stillliegt. Da die vertikale Trennwand nicht notwendigerweise die gesamte Extraktionskolonne teilt sondern insbesondere Bereiche im Sumpf und im Kopf der Extraktionskolonne nicht durch die Trennwand getrennt werden, ist auch bei Betrieb nur eines der durch die vertikale Trennwand getrennten Bereiche der Extraktionskolonne im anderen Bereich Flüssigkeit enthalten. Aus diesem Grund ist die vertikale Trennwand, wie vorstehend bereits beschrieben, so zu gestalten, dass eine Schlaufenströmung vermieden wird. Dies kann zum Beispiel erzielt werden, indem die vertikale Trennwand in die abzutrennende Flüssigphase im Sumpf der Extraktionskolonne hineinragt. Die untere Begrenzung der vertikaler Trennwand ragt dabei so tief in die abzutrennende Flüssigphase hinein, dass die unterschiedliche Lage der Phasengrenze zwischen der abzutrennenden Flüssigphase und dem Extraktionsgemisch, das alle Komponenten enthält, nicht zu einer Unter- bzw. Überströmung der vertikalen Trennwand mit der kontinuierlichen Phase führt.

Eine Umströmung der vertikalen Trennwand kann alternativ dadurch verhindert werden, dass die vertikale Trennwand unterhalb dem Flüssigkeitsspiegel in der Extraktionskolonne und oberhalb einer ersten Zufuhrvorrichtung am Kopf der Extraktionskolonne endet.

Über die erste Zufuhrvorrichtung am Kopf der Extraktionskolonne wird üblicherweise das Solvent zugeführt. Am unteren Bereich der Kolonne wird über eine zweite Zufuhrvorrichtung das zu trennende Gemisch in die Extraktionskolonne eingebracht. Das Solvent und das zu trennende Gemisch werden somit in der Extraktionskolonne im Gegenstrom geführt.

Um jeden der durch die vertikale Trennwand getrennten Bereiche unabhängig voneinander betreiben zu können, mündet in jeden der durch die vertikale Trennwand getrennten Bereiche ein erster Zulauf im oberen Bereich der Extraktionskolonne und ein zweiter Zulauf am unteren Ende der Kolonne oberhalb des Sumpfs. Durch den ersten Zulauf kann dann jeweils das Solvent und durch den zweiten Zulauf das zu trennende Gemisch zugegeben werden.

Alternativ ist es jedoch auch möglich, zum Beispiel einen gemeinsamen Verteiler für beide Bereiche vorzusehen, jedoch zum Beispiel durch Ventile jeweils den Teil des Verteilers, durch den Flüssigkeit in einen Bereich zugeführt wird, verschließen zu können und den anderen geöffnet zu halten. Auf diese Weise kann über einen gemeinsamen Verteiler jeweils entweder ein Bereich oder auch die gesamte Extraktionskolonne mit Flüssigkeit beaufschlagt werden.

Als Verteiler eignet sich dabei jeder beliebige, dem Fachmann bekannte Verteiler. Geeignete Verteiler sind zum Beispiel in Form von aus einem Hauptverteiler abzweigenden Rohrleitungen ausgebildet, wobei in den einzelnen Rohrleitungen Austrittsöffnungen vorgesehen sind, durch die die zuzuführende Flüssigkeit austreten kann. Alternativ ist es jedoch zum Beispiel auch möglich, Ringverteiler oder ähnliche, dem Fachmann bekannte Verteiler einzusetzen.

Ausführungsbeispiele der Erfindung sind in den Zeichnungen dargestellt und werden in der nachfolgenden Beschreibung näher erläutert.

Es zeigen:

Figur 1    eine erfindungsgemäß ausgebildete Extraktionskolonne,

Figur 2    ein Verfahrensfließbild des Extraktionsverfahrens,

Figur 3    eine detaillierte Darstellung des Solventzulaufs,

Figur 4    eine detaillierte Darstellung des Zulaufs des zu trennenden Flüssigkeitsgemischs,

Figur 5    eine schematische Darstellung des Verfahrens zur Herstellung des aromatischen Amins.

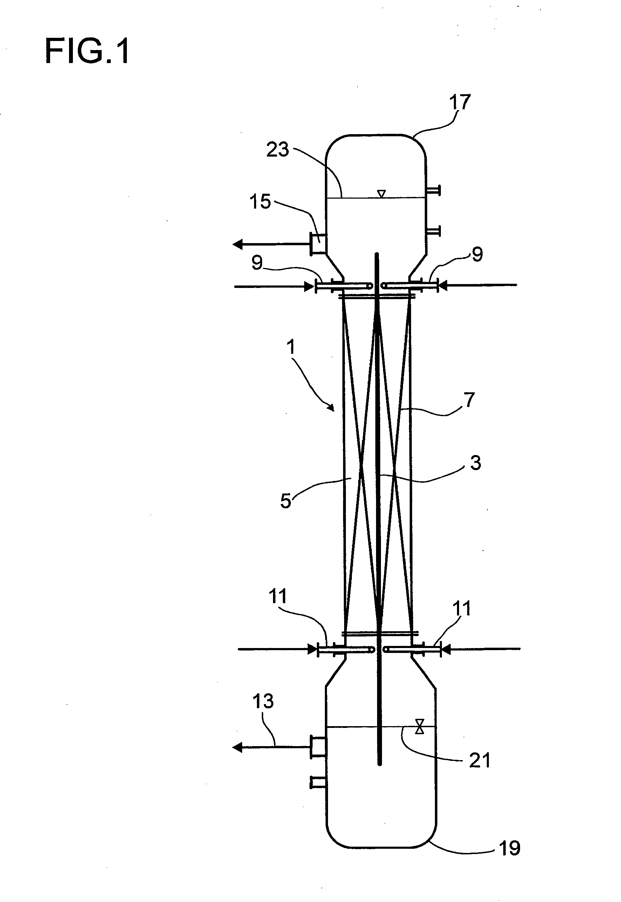

In Figur 1    ist eine erfindungsgemäß ausgebildete Extraktionskolonne dargestellt.

Eine erfindungsgemäß ausgebildete Extraktionskolonne 1 wird durch eine vertikale Trennwand 3 in einen ersten Bereich 5 und einen zweiten Bereich 7 getrennt. Der erste Bereich 5 und der zweite Bereich 7 sind dabei vorzugsweise gleich aufgebaut. Auch ist die vertikale Trennwand 3 vorzugsweise so positioniert, dass die Bereiche 5, 7 jeweils gleich groß sind.

In jeden der zwei Teilbereiche 5, 7 mündet ein erster Zulauf 9 am oberen Bereich der Extraktionskolonne 1. Durch den ersten Zulauf 9 wird das Solvent zugeführt. Um den ersten Teilbereich 5 und den zweiten Teilbereich 7 separat voneinander betreiben zu können, sind jeweils unabhängige erste Zuläufe 9 in den ersten Bereich 5 und den zweiten Bereich 7 vorgesehen. Die ersten Zuläufe 9 in den ersten Bereich 5 und den zweiten Bereich 7 sind dabei vorzugsweise gleich ausgebildet. Um den ersten Bereich 5 und den Bereich 7 unabhängig voneinander betreiben zu können, münden die ersten Zuläufe 9 unterhalb des oberen Abschlusses der vertikalen Trennwand 3 in die Extraktionskolonne 1.

Alternativ ist es jedoch auch möglich, zum Beispiel nur einen ersten Zulauf 9 zur Zufuhr des Solvents vorzusehen, jedoch muss auch mit einem einzigen ersten Zulauf 9 sichergestellt werden, dass der erste Teilbereich 5 und der zweite Bereich 7 separat voneinander betrieben werden können. Hierzu ist es in diesem Fall zum Beispiel möglich, den ersten Zulauf 9 so zu gestalten, dass durch Absperrorgane, beispielsweise Ventile, jeweils der Zulauf in den ersten Bereich 5 oder in den zweiten Bereich 7 verschlossen werden kann.

Über einen zweiten Zulauf 11 am unteren Ende der Extraktionskolonne 1 wird jedem der Bereiche 5, 7 ein zu trennendes Flüssigkeitsgemisch zugeführt. Im Rahmen der vorliegenden Erfindung ist das zu trennende Flüssigkeitsgemisch ein Gemisch, das Wasser und aromatisches Amin enthält. Der zweite Zulauf 11 ist ebenso wie der erste Zulauf 9 so ausgestaltet, dass unabhängig voneinander sowohl der erste Bereich 5 als auch der zweite Bereich 7 mit dem zu trennenden Flüssigkeitsgemisch beaufschlagt werden können. Auch ist eine Beaufschlagung beider Bereiche 5, 7 mit dem Flüssigkeitsgemisch durch den zweiten Zulauf 11 möglich. Hierzu ist es ebenso wie für den ersten Zulauf 9 möglich, dass zwei separate zweite Zuläufe 11 in jedem der Bereiche 5, 7 vorgesehen sind, alternativ ist jedoch ein einziger zweiter Zulauf 11 denkbar, bei dem jeweils die Verteiler in den ersten Bereich 5 oder in den zweiten Bereich 7 durch geeignete Absperrorgane, beispielsweise Ventile verschließbar sind, so dass entweder der erste Bereich, der zweite Bereich 7 oder beide Bereiche 5, 7 betrieben werden können.

Zur besseren Dispergierung und zur Verlängerung der Verweilzeit des zu trennenden Flüssigkeitsgemisches mit dem Solvent sind im ersten Bereich 5 und im zweiten Bereich 7 jeweils geeignete Einbauten vorgesehen. Als Einbauten eignen sich insbesondere strukturierte oder unstrukturierte Packungen, insbesondere unstrukturierte Packungen. Als unstrukturierte Packungen werden üblicherweise Füllkörper bezeichnet. Füllkörper, die im Rahmen der vorliegenden Erfindung eingesetzt werden können, sind zum Beispiel Pallringe, Raschigringe, Sättel oder beliebige andere, für die Extraktion geeignete Füllkörper, die dem Fachmann bekannt sind.

Um eine hinreichende Extraktionswirkung zu erzielen, sind die ersten Zuläufe 9 vorzugsweise oberhalb der in den Bereichen 5, 7 enthaltenen Einbauten positioniert. Die zweiten Zuläufe 11, durch die das zu trennende Flüssigkeitsgemisch zugegeben wird, sind vorzugsweise unterhalb der Einbauten in den Bereichen 5, 7 angeordnet. Um einen Betrieb der einzelnen Bereiche 5, 7 zu ermöglichen ist dabei die Trennwand so ausgebildet, dass die Zuläufe 9, 11 jeweils in die durch die Trennwand 3 getrennten Bereiche 5, 7 der Extraktionskolonne 1 münden, auch wenn die Trennwand 3, wie in Figur 1 dargestellt, nicht die gesamte Extraktionskolonne 1 teilt.

Eine vertikale Trennwand 3, die nicht die gesamte Extraktionskolonne teilt, wie beispielsweise in Figur 1 dargestellt, ist bevorzugt, da dies ermöglicht, nur einen Flüssigkeitsablauf 13 am Sumpf der Kolonne und einen zweiten Flüssigkeitsablauf 15 am Kopf der Kolonne vorzusehen. Bei einer vollständigen Trennung in zwei Bereiche wäre für jeden Bereich ein separater Flüssigkeitsablauf am Sumpf als auch am Kopf notwendig. Zudem wäre bei einer vollständigen Teilung der Extraktionskolonne 1 auch erforderlich, sämtliche notwendigen Mess- und Regeleinheiten in beiden Bereichen vorzusehen. Bei einer Trennwand 3, wie sie in Figur 1 dargestellt ist, sind jedoch am Kopf 17 und am Sumpf 19 der Extraktionskolonne 1 jeweils einfache Mess- und Regeleinheiten ausreichend.

Über den zweiten Flüssigkeitsablauf 15 am Kopf 17 der Extraktionskolonne 1 wird ein im Wesentlichen Wasser enthaltender Strom entnommen, aus dem das aromatische Amin extrahiert worden ist. Am Sumpf 19 der Extraktionskolonne 1 sammelt sich der als Solvent eingesetzte Nitroaromat mit dem darin gelösten aromatischen Amin. Zwischen der abzutrennenden Flüssigphase, die den Nitroaromaten und das aromatische Amin enthält und dem zu extrahierenden Flüssigkeitsgemisch, das zusätzlich auch das Wasser enthält, bildet sich eine Phasengrenze 21. Um einen unabhängigen Betrieb des ersten Bereichs 5 und des zweiten Bereichs 7 zu ermöglichen ist es notwendig, dass die Trennwand 3 unterhalb der Phasengrenze 21 im abzutrennenden Flüssigkeitsgemisch, das über den Flüssigkeitsablauf 13 am Sumpf 19 der Extraktionskolonne 1 abgezogen wird, endet. Die vertikale Trennwand 3 muss dabei soweit in die abzutrennende Flüssigphase hineinragen, dass bei einem Betrieb nur eines der Bereiche 5, 7 eine Schlaufenströmung vermieden wird.

Die Extraktionskolonne 1 wird üblicherweise so betrieben, dass diese nicht vollständig mit Flüssigkeit befüllt ist. Der Flüssigkeitsstand in der Extraktionskolonne 1 ist in der hier dargestellten Ausführungsform durch Bezugszeichen 23 gekennzeichnet.

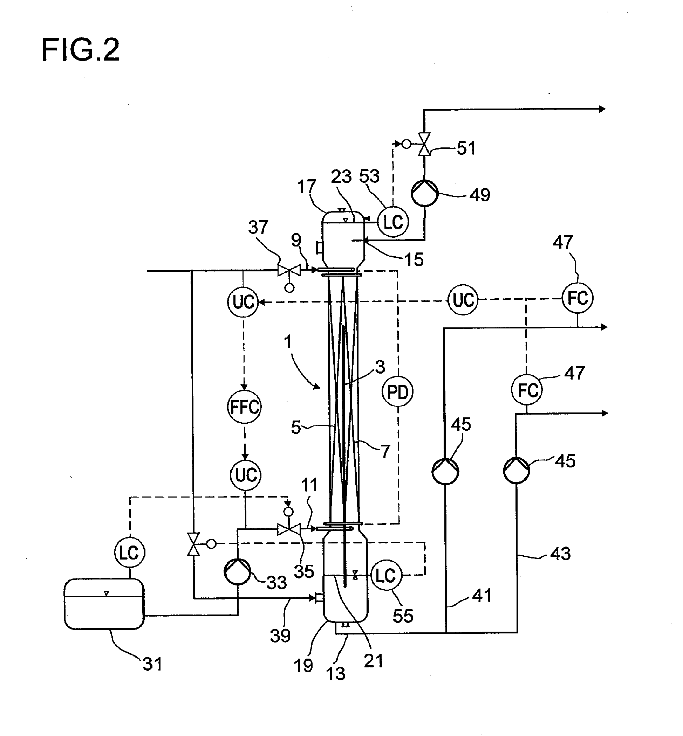

In Figur 2 ist ein Flussdiagramm des Extraktionsverfahrens dargestellt.

Ein zu trennendes, Wasser und aromatisches Amin enthaltendes Flüssigkeitsgemisch wird in einem Vorlagebehälter 31 gesammelt. Das Wasser und aromatisches Amin enthaltende Flüssigkeitsgemisch entstammt zum Beispiel einem Verfahren zur Herstellung von aromatischem Amin aus dem korrespondierenden Nitroaromaten. In dem Vorlagebehälter 31 kann das Wasser und aromatisches Amin enthaltende Flüssigkeitsgemisch aus einem oder aus mehreren Prozessen zur Herstellung des aromatischen Amins gesammelt werden. So ist es zum Beispiel möglich, das aromatisches Amin und Wasser enthaltende Flüssigkeitsgemisch aus mehreren Prozessen in nur einer Extraktionskolonne 1 aufzuarbeiten. Da in diesem Fall jedoch abhängig von der geforderten Produktionsmenge des aromatischen Amins unter Umständen nur ein Teil der Prozesse betrieben wird, ist es notwendig, die Extraktionskolonne 1 über einen großen Lastbereich betreiben zu können. Hierzu ist die Extraktionskolonne 1 mit der Trennwand 3 in zwei Bereiche 5, 7 getrennt. Auf diese Weise ist es möglich, nur einen der Bereiche 5, 7 oder beide Bereiche 5 und 7 zu betreiben. Dies erlaubt einen Betrieb der Extraktionskolonne 1 über einen sehr weiten Lastbereich, so dass auch, wenn beispielsweise zwei Prozesse zur Herstellung des aromatischen Amins vorgesehen sind, eine zufrieden stellende Trennung erreicht wird, wenn nur einer der zwei Prozesse zur Herstellung des aromatischen Amins betrieben wird. Neben dem Vorlagebehälter 31 ist es alternativ selbstverständlich auch möglich, dass die Extraktionskolonne 1 direkt mit dem aromatisches Amin und Wasser enthaltenden Flüssigkeitsgemisch aus dem Herstellungsprozess des aromatischen Amins versorgt wird.

In der hier dargestellten Ausführungsform wird das das Wasser und das aromatische Amin enthaltende Flüssigkeitsgemisch mit einer Pumpe 33 über den zweiten Zulauf 11 am unteren Bereich der Extraktionskolonne 1 in die Extraktionskolonne 1 zugeführt. Die zuzuführende Flüssigkeitsmenge kann über ein Ventil 35 gesteuert werden.

Über den ersten Zulauf 9 im oberen Bereich der Extraktionskolonne 1 wird das Solvent, insbesondere der auch zur Herstellung des aromatischen Amins eingesetzte Nitroaromat, zugeführt. Um die Menge des zuzuführenden Nitroaromaten einzustellen, ist ein Regelventil 37 vorgesehen.

Zur Überwachung des Betriebes wird die Lage der Phasengrenze 21 überwacht. Sobald die Lage der Phasengrenze 21 einen unteren Wert unterschreitet ist es möglich, über einen Zulauf 39 Solvent in den Sumpf zuzuführen. Hierdurch lässt sich die abzutrennende Flüssigkeitsmenge am Sumpf 19 der Extraktionskolonne erhöhen und die Phasengrenze 21 wird nach oben verschoben. Dies ist insbesondere bei Betrieb nur eines der Bereiche 5, 7 notwendig, um zu verhindern, dass die Phasengrenze 21 unter das untere Ende der Trennwand 3 gelangt, damit keine Schlaufenströmung entsteht.

Die abzutrennende Flüssigphase, die den Nitroaromaten als Solvent und das darin gelöste aromatische Amin enthält, wird durch den Flüssigkeitsablauf 13 am Sumpf 19 der Extraktionskolonne 1 abgezogen. In einer bevorzugten Ausführungsform wird der Extraktstrom als Edukt der Herstellung des aromatischen Amins zugeführt. In der hier dargestellten Ausführungsform wird der Flüssigkeitsablauf 13 in zwei separate Leitungen 41, 43 geteilt, wobei jede der Leitungen 41, 43 beispielsweise in einen Reaktor zur Herstellung des aromatischen Amins münden. Um die Reaktoren separat betreiben zu können ist in jeder der Leitungen 41, 43 eine Pumpe 45 aufgenommen. Mit der Pumpe 45 kann über die jeweilige Leitung 41 oder 43 der Extraktstrom in den jeweils mit der Leitung 41, 43 verbundenen Reaktor transportiert werden. Mit einem Durchflussmesser 47 wird der jeweils über die Leitung 41, 43 entnommene Eduktstrom gemessen. Auch lässt sich hierdurch die Menge des zuzuführenden Nitroaromaten regeln.

Über den zweiten Flüssigkeitsablauf 15 am Kopf 17 der Extraktionskolonne 1 wird der im Wesentlichen Wasser enthaltende Strom, aus dem das aromatische Amin extrahiert wurde, entnommen. Die Menge des über den zweiten Flüssigkeitsablauf 15 entnommenen Raffinats wird zum Beispiel durch eine Pumpe 49 und ein Regelventil 51 eingestellt. Die Pumpe 49 dient dabei insbesondere dem Transport des Raffinatstroms und das Regelventil 51 der Einstellung der zu entnehmenden Menge. Insbesondere lässt sich über die Pumpe 49 und das Regelventil 51 auch der Flüssigkeitsstand 23 in der Extraktionskolonne 1 einstellen. Der Flüssigkeitsstand 23 wird dabei mit einem geeigneten Füllstandsmesser 53 überwacht.

Aufgrund der nicht durchgehenden vertikalen Trennwand 3 ist es ausreichend, jeweils einen Füllstandsmesser 53 am Kopf der Kolonne und einen Flüssigkeitsstandsmesser 55, mit dem die Lage der Phasengrenze 21 überwacht wird, vorzusehen. Bei einer vollständigen Trennung wäre es erforderlich, in jedem der durch die vertikale Trennwand 3 getrennten Bereiche der Extraktionskolonne 1 einen solchen Füllstandsmesser 53 bzw. Flüssigkeitsstandsmesser 55 vorzusehen.

Um einen fehlerfreien Betrieb der Extraktionskolonne sicherzustellen ist es weiterhin vorteilhaft, das Durchflussverhältnis zwischen Zulauf von zu trennendem Gemisch und Solvent zu regeln. Dies erfolgt üblicherweise über die Ventile 35, 37. Auch ist es vorteilhaft, die Druckdifferenz über die Einbauten in der Extraktionskolonne 1 zu überwachen.

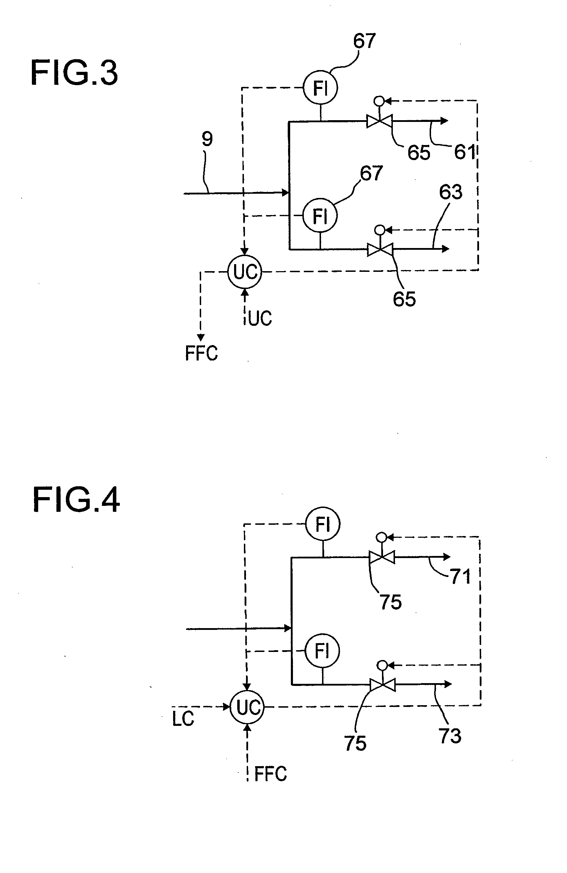

In Figur 3 ist der erste Zulauf 9, über den das Solvent zugegeben wird, detailliert dargestellt. Um den ersten Bereich 5 und den zweiten Bereich 7 der Extraktionskolonne jeweils unabhängig voneinander betreiben zu können, ist es notwendig, sowohl dem ersten Bereich 5 als auch dem zweiten Bereich 7 unabhängig voneinander Solvent zuführen zu können. Daher teilt sich der erste Zulauf 9 in einer bevorzugten Ausführungsform, die hier dargestellt ist, in einen Zulauf 61 in den ersten Bereich 5 der Extraktionskolonne 1 und einen zweiten Zulauf 63 in den zweiten Bereich 7 der Extraktionskolonne 1. Der Zulauf 61 in den ersten Bereich 5 und der Zulauf 63 in den zweiten Bereich 7 können jeweils unabhängig voneinander durch ein Ventil 65 verschlossen oder geöffnet werden. Bei geöffnetem Ventil 65 strömt Solvent durch den jeweiligen Zulauf 61, 63 in die Extraktionskolonne, bei geschlossenem Ventil 65 ist der jeweilige Zulauf geschlossen. Auf diese Weise ist es möglich unabhängig voneinander entweder den ersten Bereich 5, den zweiten Bereich 7 oder beide Bereiche gleichzeitig zu betreiben. Zur Überwachung ist in jedem der Zuläufe 61, 63 jeweils eine Flüssigkeitsanzeige 67 vorgesehen. Als Regelgröße dient unter anderem die Durchflussmenge des aus dem Prozess entnommenen Extraktstroms.

In Figur 4 ist detailliert der Zulauf des zu trennenden Flüssigkeitsgemischs dargestellt. Das zu trennende Flüssigkeitsgemisch wird über einen Zulauf 71 in den ersten Bereich der Extraktionskolonne und über einen Zulauf 73 in den zweiten Bereich der Extraktionskolonne zugeführt. In den Zuläufen 71, 73 ist jeweils ein Ventil 75 zur Einstellung der Flüssigkeitsmenge, die durch den jeweiligen Zulauf 71, 73 in die Extraktionskolonne 1 zugeführt werden soll, aufgenommen. Auch kann, wenn der Betrieb nur eines Bereiches 5 oder 7 vorgesehen ist, der Zulauf 71 oder 73, der in den nicht betriebenen Bereich 5 oder 7 der Extraktionskolonne 1 mündet, verschlossen werden. Ebenso wie für die Solventzufuhr ist auch dies notwendig, um einen unabhängigen Betrieb des ersten Bereichs 5 oder des zweiten Bereichs 7 zu ermöglichen. Die Regelung der Ventile 75 erfolgt dabei zum einen über das Durchflussverhältnis zwischen Zulauf an Solvent und Zulauf von zu trennendem Gemisch als auch den Füllstand des Vorlagebehälters 31. Um die Ventile 75 entsprechend steuern zu können, werden einem Regler für die Ventile das Durchflussverhältnis, der Durchfluss durch den jeweiligen Zulauf 71, 73 und der Flüssigkeitsstand des Vorlagebehälters 31 zugeführt.

Zur Regelung der Ventile 65, durch die das Solvent über die Zuläufe 61, 63 in die Extraktionskolonne eingeleitet wird, wird einem Regler jeweils die Durchflussmenge durch die Zuläufe 61, 63 als auch die Durchflussmenge des entnommenen Extraktstroms zugeführt. Das Durchflussverhältnis geht als Regelgröße an den Regler für den Zulauf 71, 73 für das zu trennende Flüssigkeitsgemisch.

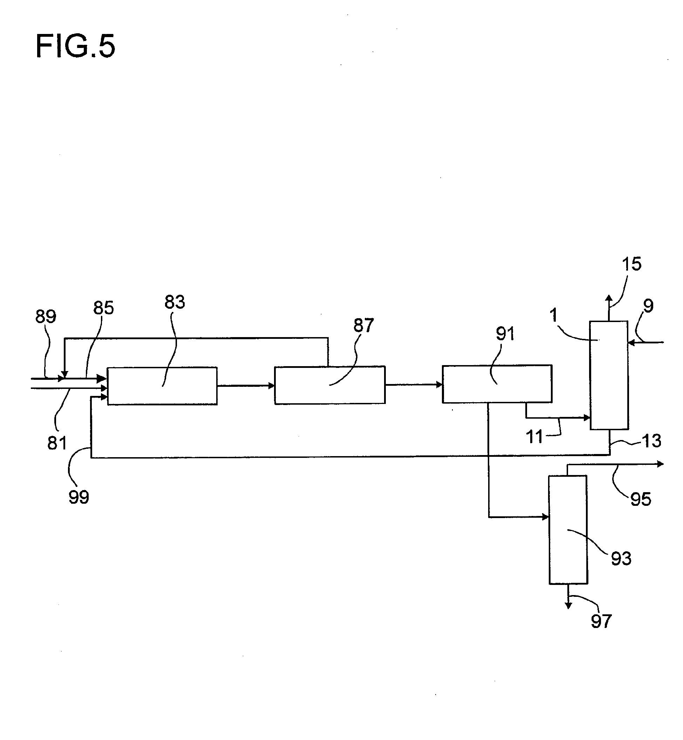

In Figur 5 ist schematisch das Verfahren zur Herstellung von aromatischem Amin dargstellt.

Über einen Eduktzulauf 81 wird einem Reaktor 83 ein Nitroaromat als Edukt zugeführt. Wenn das herzustellende aromatische Amin Anilin ist, wird als Nitroaromat Nitrobenzol eingesetzt. Über einen zweiten Zulauf 85 wird dem Reaktor 83 Wasserstoff eingespeist. Im Reaktor 83 werden der Nitroaromat und der Wasserstoff zum aromatischen Amin umgesetzt. Das entstehende Produktgemisch wird einem Kondensator 87 zugeführt. Im Kondensator 87 kondensieren das aromatische Amin und das bei der Reaktion entstehende Wasser aus. Nicht umgesetzter Wasserstoff wird über den zweiten Zulauf 85 in den Reaktor 83 zurückgeführt. Verbrauchter Wasserstoff wird über einen Wasserstoffzulauf 89 ergänzt.

Nach der Kondensation wird das verbleibende, Wasser und aromatisches Amin enthaltende Flüssigkeitsgemisch einer Entwässerungseinheit 91 zugeführt. Als Entwässerungseinheit 91 eignet sich zum Beispiel eine Flüssig/Flüssig-Phasentrennung. In der Entwässerungseinheit 91 wird das Wasser und aromatisches Amin enthaltende Flüssigkeitsgemisch in einen ersten Strom, der aromatisches Amin und Rückstände enthält, und einen zweiten Strom, der Wasser und Rückstände des aromatischen Amins enthält, getrennt.

Der aromatisches Amin und Rückstände enthaltende Strom wird einer Destillationskolonne 93 zugeführt. In der Destillationskolonne 93 wird der das aromatische Amin enthaltende Strom in einen Kopfstrom 95, der aromatisches Amin als Reinprodukt enthält, und einen Rückstände enthaltenden Sumpfstrom 97 getrennt.

Das Wasser und Rückstände des aromatischen Amins enthaltende Flüssigkeitsgemisch wird einer erfindungsgemäßen Extraktionskolonne 1 zugeführt. Die Extraktionskolonne 1 ist dabei beispielsweise aufgebaut wie in Figur 1 dargestellt. Der Extraktionskolonne 1 wird der zur Herstellung des aromatischen Amins eingesetzte Nitroaromat als Solvent über dem ersten Zulauf 9 zugeführt. Das Wasser und Rückstände des aromatischen Amins enthaltende Flüssigkeitsgemisch wird über den zweiten Zulauf 11 am unteren Bereich der Extraktionskolonne 1 zugeführt. Über den zweiten Flüssigkeitsablauf 15 am Kopf der Extraktionskolonne 1 wird ein im Wesentlichen Wasser enthaltender Raffinatstrom abgezogen und über den Flüssigkeitsablauf 13 am Sumpf der Extraktionskolonne 1 der den Nitroaromaten und das aromatische Amin enthaltende Extraktstrom entnommen. Der am Flüssigkeitsablauf 13 am Sumpf der Extraktionskolonne 1 entnommene Extraktstrom wird über einen dritten Zulauf 99 in den Reaktor 83 zurückgeführt. Durch die Rückführung des den Nitroaromaten und das aromatische Amin enthaltenden Extraktstroms in den Reaktor 83 und das weitere Durchführen des aromatischen Amins durch die übrigen Prozessschritte wird die Ausbeute an aromatischem Amin erhöht. Insbesondere ist es nicht erforderlich, den über den Flüssigkeitsablauf 13 am Sumpf der Extraktionskolonne 1 entnommenen Extraktstrom separat aufzubereiten.

<u>Bezugszeichenliste</u>

| 1 | Extraktionskolonne | 81 | Eduktzulauf |
|---|---|---|---|
| 3 | Tennwand | 83 | Reaktor |
| 5 | erster Bereich | 85 | zweiter Zulauf |
| 7 | zweiter Bereich | 87 | Kondensator |
| 9 | erster Zulauf | 89 | Wasserstoffzulauf |
| 11 | zweiter Zulauf | 91 | Entwässerungseinheit |
| 13 | Flüssigkeitsablauf am Sumpf | 93 | Destillationskolonne |
| 15 | zweiter Flüssigkeitsablauf | 95 | Kopfstrom |
| 17 | Kopf | 97 | Sumpfstrom |
| 19 | Sumpf | 99 | dritter Zulauf |
| 21 | Phasengrenze | | |

(fortgesetzt)

| | |
|---|---|
| 23 | Flüssigkeitsstand |
| 31 | Vorlagebehälter |
| 33 | Pumpe |
| 35 | Ventil |
| 37 | Regelventil |
| 39 | Zulauf |
| 41 | Leitung |
| 43 | Leitung |
| 45 | Pumpe |
| 47 | Durchflussmesser |
| 49 | Pumpe |
| 51 | Regelventil |
| 53 | Füllstandsmesser |
| 55 | Flüssigkeitsstandsmesser |
| 61 | Zulauf im ersten Bereich |
| 63 | Zulauf im zweiten Bereich |
| 65 | Ventil |
| 67 | Flüssigkeitsanzeige |
| 71 | Zulauf im ersten Bereich |
| 73 | Zulauf im zweiten Bereich |
| 75 | Ventil |

**Patentansprüche**

1. Verfahren zur Gewinnung mindestens eines aromatischen Amins aus einem Wasser und das mindestens eine aromatische Amin enthaltenden Flüssigkeitsgemisch durch Extraktion mit mindestens einem Nitroaromaten in einer Extraktionskolonne (1), wobei ein mindestens 98 Gew.-% Wasser enthaltender Raffinatstrom und ein den mindestens einen Nitroaromaten und das aromatische Amin enthaltender Extraktstrom gebildet werden, **dadurch gekennzeichnet, dass** die Extraktionskolonne (1) durch eine vertikale Trennwand (3) in zwei Bereiche (5, 7) getrennt wird, wobei bei einer zu trennenden Flüssigkeitsmenge, die kleiner ist als eine minimale Querschnittsbejastung der gesamten Extraktionskolonne (1), nur einem der durch die vertikale Trennwand (3) getrennten Bereiche (5, 7) der Extraktionskolonne (1) das zu trennende Flüssigkeitsgemisch zugeführt wird.

2. Verfahren gemäß Anspruch 1, **dadurch gekennzeichnet, dass** die vertikale Trennwand (3) unterhalb dem Flüssigkeitsspiegel (23) in der Extraktionskolonne (1) und oberhalb einer ersten Zufuhrvorrichtung (9) am Kopf (17) der Extraktionskolonne (1) endet.

3. Verfahren gemäß Anspruch 1 oder 2, **dadurch gekennzeichnet, dass** die vertikale Trennwand (3) in die abzutrennende Flüssigphase im Sumpf (19) der Extraktionskolonne (1) hineinragt.

4. Verfahren gemäß einem der Ansprüche 1 bis 3, **dadurch gekennzeichnet, dass** die abzutrennende Flüssigphase im Sumpf (19) der Extraktionskolonne (1) eine den mindestens einen Nitroaromaten und das mindestens eine aromatische Amin und maximal 1 Gew.-% einer Fremdphase enthaltende Extraktphase ist und am Kopf (17) der Extraktionskolonne (1) ein mindestens 98 Gew.-% Wasser enthaltender Raffinatstrom entnommen wird.

5. Verfahren gemäß einem der Ansprüche 1 bis 4, **dadurch gekennzeichnet, dass** in jeden der durch die vertikale Trennwand (3) getrennten Bereiche (5, 7) ein erster Zulauf (9) im oberen Bereich der Extraktionskolonne (1) und ein zweiter Zulauf (11) am unteren Ende der Extraktionskolonne (1) oberhalb des Sumpfs (19) münden.

6. Verfahren gemäß Anspruch 5, **dadurch gekennzeichnet, dass** über den ersten Zulauf (9) im oberen Bereich der

**EP 2 427 424 B1**

Extraktionskolonne (1) der mindestens eine Nitroaromat und über den zweiten Zulauf (11) das zu trennende das Wasser und das mindestens eine aromatische Amin enthaltende Flüssigkeitsgemisch zugegeben werden.

7. Verfahren gemäß einem der Ansprüche 1 bis 6, **dadurch gekennzeichnet, dass** der Nitroaromat Nitrobenzol und das aromatische Amin Anilin ist.

8. Verfahren zur Herstellung von aromatischen Aminen durch katalytische Hydrierung des korrespondierenden Nitroaromaten, folgende Schritte umfassend:

   (a) Umsetzen eines den Nitroaromaten und Wasserstoff enthaltenden Reaktionsgemischs in Gegenwart eines heterogenen Katalysators in einem Reaktor (83) zu einem das aromatische Amin, Wasser und nicht umgesetzten Wasserstoff enthaltenden Produktgemisch in einer Gleichgewichtsreaktion,
   (b) Entfernen des Wasserstoffs aus dem Produktgemisch,
   (c) Entwässern des Produktgemischs, wobei ein maximal 1 Gew.-% einer Fremdphase enthaltendes aromatisches Amin als Produkt und ein Wasser und Rückstände des aromatischen Amins enthaltendes Flüssigkeitsgemisch erhalten werden,
   (d) Trennen des in Schritt (c) erhaltenen Wasser und Rückstände des aromatischen Amins enthaltenden Flüssigkeitsgemischs in einem Verfahren gemäß einem der Ansprüche 1 bis 7 durch Extraktion mit einem Nitroaromaten in einen mindestens 98 Gew.-% Wasser enthaltenden Raffinatstrom und einen den Nitroaromaten und das aromatische Anilin enthaltenden Extraktstrom.

9. Verfahren gemäß Anspruch 8, **dadurch gekennzeichnet, dass** der in Schritt (d) als Solvent eingesetzte Nitroaromat der zur Herstellung des aromatischen Amins eingesetzte Nitroaromat ist.

10. Verfahren gemäß Anspruch 9, **dadurch gekennzeichnet, dass** der in Schritt (d) erhaltene Extraktstrom in den Reaktor (83) zurückgeführt wird.

11. Verfahren gemäß einem der Ansprüche 8 bis 10, **dadurch gekennzeichnet, dass** das in Schritt (c) erhaltene, das aromatische Amin enthaltende Produkt durch eine Destillation von Rückständen befreit wird.

12. Vorrichtung zur Trennung eines Wasser und mindestens ein aromatisches Amin enthaltenden Flüssigkeitsgemischs durch Extraktion mit mindestens einem Nitroaromaten, umfassend eine Extraktionskolonne (1), wobei die Extraktionskolonne (1) durch eine vertikale Trennwand (3) in zwei Bereiche (5, 7) getrennt wird, und weiterhin Mittel zur Steuerung vorgesehen sind, mit denen die Extraktion so geregelt werden kann, dass bei einer zu trennenden Flüssigkeitsmenge, die kleiner ist als die minimale Querschnittsbelastung der gesamten Extraktionskolonne (1), nur einem der durch die vertikale Trennwand (3) getrennten Bereiche (5, 7) der Extraktionskolonne (1) das zu trennende Flüssigkeitsgemisch zugeführt wird.

13. Vorrichtung gemäß Anspruch 12, **dadurch gekennzeichnet, dass** die vertikale Trennwand (3) unterhalb dem Flüssigkeitsspiegel (23) in der Extraktionskolonne (1) und oberhalb einer ersten Zufuhrvorrichtung am Kopf der Extraktionskolonne endet.

14. Vorrichtung gemäß Anspruch 12 oder 13, **dadurch gekennzeichnet, dass** die vertikale Trennwand (3) in die abzutrennende Flüssigphase im Sumpf (19) der Extraktionskolonne (1) hineinragt.

15. Vorrichtung gemäß einem der Ansprüche 12 bis 14, **dadurch gekennzeichnet, dass** in jeden der durch die vertikale Trennwand (3) getrennten Bereiche (5, 7) ein erster Zulauf (9) im oberen Bereich der Extraktionskolonne (1) und ein zweiter Zulauf (11) am unteren Ende der Extraktionskolonne (1) oberhalb des Sumpfs (19) mündet.

**Claims**

1. A process for obtaining at least one aromatic amine from a liquid mixture comprising water and the at least one aromatic amine by extracting with at least one nitroaromatic in an extraction column (1) to form a raffinate stream comprising at least 98% by weight of water and an extract stream comprising the at least one nitroaromatic and the aromatic amine, wherein the extraction column (1) is divided by a vertical dividing wall (3) into two regions (5, 7), and, in the case of an amount of liquid for separation which is less than a minimum superficial velocity of the entire extraction column (1), the liquid mixture to be separated is fed only to one of the regions (5, 7) of the extraction

**12**

column (1) divided by the vertical dividing wall (3).

2. The process according to claim 1, wherein the vertical dividing wall (3) ends below the liquid level (23) in the extraction column (1) and above a first feed apparatus (9) at the top (17) of the extraction column (1).

3. The process according to claim 1 or 2, wherein the vertical dividing wall (3) projects into the liquid phase to be removed in the bottom (19) of the extraction column (1).

4. The process according to any of claims 1 to 3, wherein the liquid phase to be removed in the bottom (19) of the extraction column (1) is an extract phase comprising the at least one nitroaromatic and the at least one aromatic amine and not more than 1% by weight of an extraneous phase, and a raffinate stream comprising at least 98% by weight of water is withdrawn at the top (17) of the extraction column (1).

5. The process according to any of claims 1 to 4, wherein a first feed (9) opens into the upper region of the extraction column (1) and a second feed (11) opens at the lower end of the extraction column (1) above the bottom (19) in each of the regions (5, 7) divided by the vertical dividing wall (3).

6. The process according to claim 5, wherein the at least one nitroaromatic is added via the first feed (9) in the upper region of the extraction column (1), and the liquid mixture which comprises the water and the at least one aromatic amine and is to be separated is added via the second feed (11).

7. The process according to any of claims 1 to 6, wherein the nitroaromatic is nitrobenzene and the aromatic amine is aniline.

8. A process for preparing aromatic amines by catalytically hydrogenating the corresponding nitroaromatic, comprising the following steps:

(a) converting a reaction mixture comprising the nitroaromatic and hydrogen in the presence of a heterogeneous catalyst in a reactor (83) to give a product mixture comprising the aromatic amine, water and unconverted hydrogen in an equilibrium reaction,
(b) removing the hydrogen from the product mixture,
(c) dewatering the product mixture to obtain an aromatic amine comprising not more than 1% by weight of an extraneous phase as the product, and a liquid mixture comprising water and residues of the aromatic amine,
(d) separating the liquid mixture which comprises water and residues of the aromatic amine and is obtained in step (c) in a process according to any of claims 1 to 7 by extracting with a nitroaromatic to give a raffinate stream comprising at least 98% by weight of water and an extract stream comprising the nitroaromatic and the aromatic aniline.

9. The process according to claim 8, wherein the nitroaromatic used as the solvent in step (d) is the nitroaromatic used to prepare the aromatic amine.

10. The process according to claim 9, wherein the extract stream obtained in step (d) is recycled into the reactor (83).

11. The process according to any of claims 8 to 10, wherein the product which comprises the aromatic amine and is obtained in step (c) is freed of residues by a distillation.

12. An apparatus for separating a liquid mixture comprising water and at least one aromatic amine by extracting with at least one nitroaromatic, comprising an extraction column (1), which extraction column (1) is divided by a vertical dividing wall (3) into two regions (5, 7), and control means are additionally provided, with which the extraction can be regulated such that, in the case of an amount of liquid for separation which is less than the minimum superficial velocity of the entire extraction column (1), the liquid mixture to be separated is fed only to one of the regions (5, 7) of the extraction column (1) divided by the vertical dividing wall (3).

13. The apparatus according to claim 12, wherein the vertical dividing wall (3) ends below the liquid level (23) in the extraction column (1) and above a first feed apparatus at the top of the extraction column.

14. The apparatus according to claim 12 or 13, wherein the vertical dividing wall (3) projects into the liquid phase to be removed in the bottom (19) of the extraction column (1).

**15.** The apparatus according to any of claims 12 to 14, wherein a first feed (9) opens into the upper region of the extraction column (1) and a second feed (11) opens at the lower end of the extraction column (1) above the bottom (19) in each of the regions (5, 7) divided by the vertical dividing wall (3).

**Revendications**

**1.** Procédé pour l'obtention d'au moins une amine aromatique à partir d'un mélange liquide contenant de l'eau et ladite au moins une amine aromatique par extraction avec au moins un composé nitroaromatique dans une colonne d'extraction (1), un flux de raffinat contenant au moins 98% en poids d'eau et un flux d'extrait contenant ledit au moins un composé nitroaromatique et l'amine aromatique étant formés, **caractérisé en ce que** la colonne d'extraction (1) est séparée par une paroi de séparation verticale (3) en deux zones (5, 7), où dans le cas d'une quantité de liquide à séparer qui est inférieure à une charge transversale minimale de la colonne d'extraction (1) dans sa totalité, seule une des zones (5, 7) séparées par la paroi de séparation verticale (3) de la colonne d'extraction (1) est alimentée en mélange liquide à séparer.

**2.** Procédé selon la revendication 1, **caractérisé en ce que** la paroi de séparation verticale (3) se termine sous le niveau de liquide (23) dans la colonne d'extraction (1) et au-dessus d'un premier dispositif d'alimentation (9) en tête (17) de la colonne d'extraction (1).

**3.** Procédé selon la revendication 1 ou 2, **caractérisé en ce que** la paroi de séparation verticale (3) pénètre dans la phase liquide à séparer dans le fond (19) de la colonne d'extraction (1).

**4.** Procédé selon l'une quelconque des revendications 1 à 3, **caractérisé en ce que** la phase liquide à séparer dans le fond (19) de la colonne d'extraction (1) est une phase d'extrait, contenant ledit au moins un composé nitroaromatique et ladite au moins une amine aromatique et au maximum 1% en poids d'une phase étrangère, et un flux de raffinat contenant au moins 98% en poids d'eau est prélevé en tête (17) de la colonne d'extraction (1).

**5.** Procédé selon l'une quelconque des revendications 1 à 4, **caractérisé en ce qu'**une première alimentation (9) débouche dans la partie supérieure de la colonne d'extraction (1) et une deuxième alimentation (11) débouche dans l'extrémité inférieure de la colonne d'extraction (1) au-dessus du fond (19) dans chacune des zones (5, 7) séparées par la paroi de séparation verticale (3).

**6.** Procédé selon la revendication 5, **caractérisé en ce que** ledit au moins un composé nitroaromatique est ajouté via la première alimentation (9) dans la partie supérieure de la colonne d'extraction (1) et le mélange liquide à séparer contenant l'eau et ladite au moins une amine aromatique est ajouté via la deuxième alimentation (11).

**7.** Procédé selon l'une quelconque des revendications 1 à 6, **caractérisé en ce que** le composé nitroaromatique est le nitrobenzène et l'amine aromatique est l'aniline.

**8.** Procédé pour la préparation d'amines aromatiques par hydrogénation catalytique du composé nitroaromatique correspondant, comprenant les étapes suivantes :

(a) transformation d'un mélange réactionnel, contenant le composé nitroaromatique et de l'hydrogène, en présence d'un catalyseur hétérogène dans un réacteur (83) en un mélange de produits contenant l'amine aromatique, l'eau et l'hydrogène non transformé dans une réaction d'équilibre,
(b) élimination de l'hydrogène du mélange de produits,
(c) déshydratation du mélange de produits, en obtenant une amine aromatique contenant au maximum 1% en poids d'une phase étrangère comme produit et un mélange liquide contenant de l'eau et des résidus de l'amine aromatique,
(d) séparation du mélange liquide contenant de l'eau et des résidus de l'amine aromatique obtenu dans l'étape (c) dans un procédé selon l'une quelconque des revendications 1 à 7 par extraction avec un composé nitroaromatique en un flux de raffinat, contenant au moins 98% en poids d'eau, et un flux d'extrait contenant le composé nitroaromatique et l'aniline aromatique.

**9.** Procédé selon la revendication 8, **caractérisé en ce que** le composé nitroaromatique utilisé comme solvant dans l'étape (d) est le composé nitroaromatique utilisé pour la préparation de l'amine aromatique.

**10.** Procédé selon la revendication 9, **caractérisé en ce que** le flux d'extrait obtenu dans l'étape (d) est recyclé dans le réacteur (83).

**11.** Procédé selon l'une quelconque des revendications 8 à 10, **caractérisé en ce que** le produit obtenu dans l'étape (c), contenant l'amine aromatique, est libéré de résidus par distillation.

**12.** Dispositif pour la séparation d'un mélange liquide contenant de l'eau et au moins une amine aromatique par extraction avec au moins un composé nitroaromatique, comprenant une colonne d'extraction (1), la colonne d'extraction (1) étant séparée par une paroi de séparation verticale (3) en deux zones (5, 7), et en outre des moyens pour la commande, permettant de réguler l'extraction de manière telle que dans le cas d'une quantité de liquide à séparer qui est inférieure à une charge transversale minimale de la colonne d'extraction (1) dans sa totalité, seule une des zones (5, 7) séparées par la paroi de séparation verticale (3) de la colonne d'extraction (1) est alimentée en mélange liquide à séparer.

**13.** Dispositif selon la revendication 12, **caractérisé en ce que** la paroi de séparation verticale (3) se termine sous le niveau de liquide (23) dans la colonne d'extraction (1) et au-dessus d'un premier dispositif d'alimentation en tête de la colonne d'extraction.

**14.** Dispositif selon la revendication 12 ou 13, **caractérisé en ce que** la paroi de séparation verticale (3) pénètre dans la phase liquide à séparer dans le fond (19) de la colonne d'extraction (1).

**15.** Dispositif selon l'une quelconque des revendications 12 à 14, **caractérisé en ce qu'**une première alimentation (9) débouche dans la partie supérieure de la colonne d'extraction (1) et une deuxième alimentation (11) débouche dans l'extrémité inférieure de la colonne d'extraction (1) au-dessus du fond (19) dans chacune des zones (5, 7) séparées par la paroi de séparation verticale (3).

# FIG.1

# FIG.2

## FIG.3

## FIG.4

# FIG.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0507118 B
- DE 102006008000 A
- GB 2352715 A
- GB 666544 A